# EUROPEAN PATENT APPLICATION

(11) **EP 4 043 484 A1**
(43) Date of publication of application: **17.08.2022**
(21) Application number: 20873301.4
(22) Date of filing: 30.09.2020
(51) Int. Cl.: C07K 14/705, C12N 5/10, C12N 1/15, C12N 1/19, C12N 1/21, C12N 15/12, G01N 33/15, G01N 33/50

(54) **SOMATOSTATIN RECEPTOR**

(30) Priority: 30.09.2019 JP 2019179597
(71) Applicant: Riken Bio Co., Ltd., Tokyo 170-0013 (JP)
(72) Inventor: SAIDO, Takaomi, Tokyo 170-0013 (JP); Saito, Takashi, Tokyo 170-0013 (JP); MATSUBA, Shoko, Tokyo 170-0013 (JP); TSUBUKI, Satoshi, Tokyo 170-0013 (JP); MATSUBA, Yukio, Tokyo 170-0013 (JP); ARAI, Hiroki, Wako-shi, Saitama 351-0106 (JP); MIHIRA, Naomi, Wako-shi, Saitama 351-0106 (JP); TAKAMURA, Risa, Wako-shi, Saitama 351-0106 (JP)
(74) Representative: Script IP Limited
(86) International application number: PCT/JP2020/037086
(87) International publication number: WO 2021/065985

(57) **Abstract**

An object of the present invention is to demonstrate the existence of a heterodimer of somatostatin receptor subtype 1 (SSTR1) and somatostatin receptor subtype 4 (SSTR4) and also to provide a method for screening a factor that binds to such a heterodimer and the heterodimer that can be used in the screening method. A transformant expressing a heterodimer of somatostatin receptor subtype 1 and somatostatin receptor subtype 4, into which DNA encoding somatostatin receptor subtype 1 and DNA encoding somatostatin receptor subtype 4 have been introduced. A method for screening a compound that interacts with a heterodimer of somatostatin receptor subtype 1 and somatostatin receptor subtype 4, comprising selecting the compound that interacts with the heterodimer from test compounds.

SEQUENCE LISTING

<110>  RIKEN BIO Co. Ltd.

<120>  Somatostatin receptor

<130>  2020C12981

<150>  JP 2019-179597
<151>  2019-09-30

<160>  2

<170>  PatentIn version 3.1

<210>  1
<211>  1200
<212>  DNA
<213>  Homo sapiens

<220>
<221>  misc_feature
<223>  Inventor:SAIDO, Takaomi; Saito, Takashi; MATSUBA, Shoko; TSUBUKI,
        Satoshi; MATSUBA, Yukio; ARAI, Hiroki; MIHIRA, Naomi; TAKAMURA,
       Risa

<400>  1
atgttcccca atggcaccgc ctcctctcct tcctcctctc ctagccccag cccgggcagc      60

tgcggcgaag gcggcggcag caggggcccc ggggccggcg ctgcggacgg catggaggag     120

ccagggcgaa atgcgtccca gaacgggacc ttgagcgagg gccagggcag cgccatcctg     180

atctctttca tctactccgt ggtgtgcctg gtggggctgt gtgggaactc tatggtcatc     240

tacgtgatcc tgcgctatgc caagatgaag acggccacca acatctacat cctaaatctg     300

gccattgctg atgagctgct catgctcagc gtgcccttcc tagtcacctc cacgttgttg     360

cgccactggc ccttcggtgc gctgctctgc cgcctcgtgc tcagcgtgga cgcggtcaac     420

atgttcacca gcatctactg tctgactgtg ctcagcgtgg accgctacgt ggccgtggtg     480

catcccatca aggcggcccg ctaccgccgg cccaccgtgg ccaaggtagt aaacctgggc     540

gtgtgggtgc tatcgctgct cgtcatcctg cccatcgtgg tcttctctcg caccgcggcc     600

aacagcgacg gcacggtggc ttgcaacatg ctcatgccag agcccgctca acgctggctg     660

gtgggcttcg tgttgtacac atttctcatg ggcttcctgc tgcccgtggg ggctatctgc     720

ctgtgctacg tgctcatcat tgctaagatg cgcatggtgg ccctcaaggc cggctggcag     780

cagcgcaagc gctcggagcg caagatcacc ttaatggtga tgatggtggt gatggtgttt     840

gtcatctgct ggatgccttt ctacgtggtg cagctggtca acgtgtttgc tgagcaggac     900

gacgccacgg tgagtcagct gtcggtcatc ctcggctatg ccaacagctg cgccaacccc     960

atcctctatg gctttctctc agacaacttc aagcgctctt tccaacgcat cctatgcctc    1020

agctggatgg acaacgccgc ggaggagccg gttgactatt acgccaccgc gctcaagagc    1080

cgtgcctaca gtgtggaaga cttccaacct gagaacctgg agtccggcgg cgtcttccgt 1140

aatggcacct gcacgtcccg gatcacgacg ctcgattaca aggacgacga cgataagtga 1200

<210> 2
<211> 1197
<212> DNA
<213> Homo sapiens

<400> 2
atgagcgccc cctcgacgct gcccccgggg ggcgaggaag ggctggggac ggcctggccc 60

tctgcagcca atgccagtag cgctccggcg gaggcggagg aggcggtggc ggggcccggg 120

gacgcgcggg cggcgggcat ggtcgctatc cagtgcatct acgcgctggt gtgcctggtg 180

gggctggtgg gcaacgccct ggtcatcttc gtgatccttc gctacgccaa gatgaagacg 240

gctaccaaca tctacctgct caacctggcc gtagccgacg agctcttcat gctgagcgtg 300

cccttcgtgg cctcgtcggc cgccctgcgc cactggccct tcggctccgt gctgtgccgc 360

gcggtgctca gcgtcgacgg cctcaacatg ttcaccagcg tcttctgtct caccgtgctc 420

agcgtggacc gctacgtggc cgtggtgcac cctctgcgcg cggcgaccta ccggcggccc 480

agcgtggcca agctcatcaa cctgggcgtg tggctggcat ccctgttggt cactctcccc 540

atcgccatct tcgcagacac cagaccggct cgcggcggcc aggccgtggc ctgcaacctg 600

cagtggccac acccggcctg gtcggcagtc ttcgtggtct acactttcct gctgggcttc 660

ctgctgcccg tgctggccat tggcctgtgc tacctgctca tcgtgggcaa gatgcgcgcc 720

gtggccctgc gcgctggctg gcagcagcgc aggcgctcgg agaagaaaat caccaggctg 780

gtgctgatgg tcgtggtcgt ctttgtgctc tgctggatgc ctttctacgt ggtgcagctg 840

ctgaacctct tcgtgaccag ccttgatgcc accgtcaacc acgtgtccct tatccttagc 900

tatgccaaca gctgcgccaa ccccattctc tatggcttcc tctccgacaa cttccgccga 960

ttcttccagc gggttctctg cctgcgctgc tgcctcctgg aaggtgctgg aggtgctgag 1020

gaggagcccc tggactacta tgccactgct ctcaagagca aaggtggggc agggtgcatg 1080

tgccccccac tcccctgcca gcaggaagcc ctgcaaccag aacccggccg caagcgcatc 1140

cccctcacca ggaccaccac cttcgaacaa aaactaatat cagaagaaga cctatga 1197

## Description

### Technical Field

The present invention relates to a transformant expressing a heterodimer of somatostatin receptor subtypes 1 and 4, an isolated heterodimer, and a method for screening a compound that interacts with the heterodimer.

### Background Art

Amyloid β (Aβ) deposits in brain parenchyma and blood vessels of patients with neurological diseases such as Alzheimer's disease and is regarded as a causative substance of various symptoms, including cognitive decline. Although Aβ is a physiological protein that is constantly produced in the normal brain, it is prone to aggregation and deposition and is also a major component of senile plaques found in the brain of patients with Alzheimer's disease and, Down's syndrome as well as of the elderly. Aβ consists of 39 to 43 amino acids derived from transmembrane precursor protein (βAPP) and is said to result from normal metabolic processing of βAPP in the neurons. Since the accumulation of Aβ in the brain of patients with Alzheimer's disease is considered to be caused by a defect in the Aβ catabolic system or an abnormality in the anabolic mechanism, substances involved in Aβ catabolism (degradation) are considered useful in treatment and prevention of neurological diseases such as Alzheimer's disease.

Recently, it was revealed that neprilysin, a type of neutral endopeptidase, functions as a degrading enzyme of Aβ in the brain (Non-patent Document 1), and it was reported that early accumulation of Aβ oligomers in the brain and cognitive dysfunction appears in a mouse model of Alzheimer's disease deficient in neprilysin (Non-patent Document 2). Since then, it has been reported that agerelated accumulation of Aβ in the human brain and progression of Alzheimer's disease are associated with decreased neprilysin levels (Non-patent Documents 3 and 4).

Furthermore, it has been recently revealed that a peptide hormone called somatostatin (SST), originally discovered as an inhibitor of growth hormone secretion, is involved in the activation of neprilysin (Non-patent Document 3). SST is produced in the hypothalamus of the brain, pancreatic islets of Langerhans D cells, endocrine cells (D cells) of the gastrointestinal tract, and the like, and is known as a neurotransmitter in addition to having functions, such as inhibition of growth hormone secretion, inhibition of insulin and glucagon secretion, and inhibition of secretin and gastric acid secretion. After biosynthesis, SST is converted through posttranslational processing into SST-14 consisting of 14 peptides in the pancreas and hypothalamus and SST-28 consisting of 28 peptides in the gastrointestinal tract and is known to exert its effects via a somatostatin receptor (SSTR), a member of G protein-binding receptor family.

SSTRs include subtypes 1 to 5 (SSTR1 to SSTR5), and each subtype is known to exhibit a different distribution in various tissues in the body. In particular, it has been revealed that SSTR4 is expressed at high levels in the hippocampus, the center of memory, and in the cerebral cortex, where high-level brain activity occurs, and recent studies using knockout mice have shown that SSTR1 and/or SSTR4 are potential targets for the development of preventive/therapeutic agents for Alzheimer's disease (Non-patent Document 3). SSTR1 to SSTR5 are considered to regulate diverse signal transduction pathways by forming homodimers and/or heterodimers on the cell membrane. From detailed experimental results obtained using fluorescence resonance energy transfer method, immunohistochemistry, Western blotting, and immunoprecipitation, it was reported that human SSTR4 and human SSTR5 form heterodimers, but there is no heterodimer consisting of human SSTR1 and human SSTR4 (Non-patent Document 5). In contrast, in 2019, it was reported that SSTR1 and SSTR4 may form dimers, based on the results of immunoprecipitation-Western blotting (IP-Western) using human breast cancer cell lines with SSTR1 and SSTR4 over-expressed (Non-patent Document 6). However, the IP-Western method used in Non-patent Document 6 is a method with low specificity and quantitative performance, and the Western blot analysis does not include a necessary molecular weight marker, and the like, so the experimental results in Non-patent Document 6 are not considered reliable. Non-patent Document 6 also discloses that heterodimer formation of SSTR1 and SSTR4 is further promoted in the presence of a certain SST analog. As mentioned above, there have been conflicting reports on whether SSTR1 and SSTR4 form a heterodimer, and no consensus has yet been reached.

As described above, SST is also considered to activate neprilysin via an SSTR and degrade Aβ. However, the biological half-life of SST is so short that it is not practical to use SST itself as a pharmaceutical agent. Therefore, thiourea derivatives having an affinity for SSTR4 (Patent Documents 1 and 2) and pyrrolidine derivatives (Patent Document 3) are disclosed as non-peptide SSTR agonists. In addition, Patent Document 4 describes a novel cycloalkane derivative having selective agonist activity of SSTR4, and the use of the cycloalkane derivative as a therapeutic agent and/or a preventive agent for Aβ-related neurodegenerative diseases such as Alzheimer's disease. Besides, Patent Document 5 reveals that a sulfonylamino-RF amide compound binds to SSTR1 and especially SSTR4 and discloses that the sulfonylamino-RF amide compound is useful in prevention or treatment of neuro-mutational diseases including Alzheimer's disease.

### Prior Art Documents

### Patent Documents

Patent Document 1: International Publication No. WO 1997/043278
Patent Document 2: International Publication No. WO 2011/047165
Patent Document 3: International Publication No. WO 2010/059922
Patent Document 4: Japanese unexamined Patent Application Publication No. 2015-54844
Patent Document 5: Japanese unexamined Patent Application Publication (Translation of PCT Application) No. 2007-507472

### Non-patent Documents

Non-patent Document 1: Iwata N et al., Nature Med. 6:143-151 (2000)
Non-patent Document 2: Iwata N et al., Science. 292:1550-1552 (2001)
Non-patent Document 3: Saido, Takaomi. Alzheimer-byo wa Naoseru Yobou Dekiru (phonetic spelling) (Alzheimer's Disease Can Be Cured and Prevented). SHUEISHA Inc., 2016
Non-patent Document 4: Nordberg et al., Neurobiology of Aging. 29:210-221 (2008)
Non-patent Document 5: Somvanshi et al., Cellular Signalling. 21: 1396-1414 (2009).
Non-patent Document 6: Zou et al., Oncology Letters. 17: 1723-1731(2019)

### Summary of the Invention

### Object to be Solved by the Invention

An object of the present invention is to demonstrate the existence of a heterodimer of SSTR1 and SSTR4, and also to provide a method for screening a factor that binds to such a heterodimer and the heterodimer that can be used in the screening method, thereby enabling a search for preventive and/or therapeutic agents for diseases caused by Aβ deposition such as Alzheimer's disease.

### Means to Solve the Object

The present inventors prepared CHO cells coexpressing SSTR1 and SSTR4 proteins and demonstrated that the SSTR1 and SSTR4 proteins form a heterodimer on the cell membrane by using the latest method which enables accurate detection of a protein-protein interaction called proximity ligation assay, and thus completed the present invention.

In other words, the present invention relates to (1) a transformant expressing a heterodimer of SSTR1 and SSTR4 into which a DNA encoding SSTR1 and a DNA encoding SSTR4 have been introduced; (2) the transformant according to (1) above, wherein a vector comprising the DNA encoding SSTR1 and a vector comprising the DNA encoding SSTR4 have been co-introduced; (3) the transformant according to (1) or (2) above, wherein expression of the heterodimer of SSTR1 and SSTR4 is confirmed by expressing SSTR1 and SSTR4 which have been each differently labeled in the transformant, and determining a proximity between the different labels by a proximity ligation assay method; (4) the transformant according to any one of (1) to (3) above, for elucidating a signal transduction system involving the heterodimer of SSTR1 and SSTR4; and (5) an isolated heterodimer of SSTR1 and SSTR4.

The present invention also relates to (6) a method for screening a compound that interacts with a heterodimer of SSTR1 and SSTR4, comprising selecting the compound that interacts with the heterodimer from test compounds; (7) the method according to (6) above, comprising steps (a), (b), and (c): (a) a step of bringing a test compound into contact with a heterodimer of SSTR1 and SSTR4; (b) a step of measuring interaction of the heterodimer with the test compound; and (c) a step of selecting a compound that interacts with the heterodimer based on the measurement results in (b) above; (8) the method according to (7) above, comprising steps (d), (e), and (f) after step (c): (d) a step of bringing the test compound selected in (c) above into contact with a homodimer of SSTR1 and/or a homodimer of SSTR4; (e) a step of measuring interaction of the test compound with the homodimer; and (f) a step of selecting a compound having a weaker interaction than the interaction measured in (b) above based on the measurement results in (e) above; (9) the method according to any one of (6) to (8) above, wherein the compound is an agonist or allosteric modulator of the heterodimer of SSTR1 and SSTR4, (10) the method according to any one of (6) to (8) above; wherein the compound is a compound used to identify an organ, tissue, or cell expressing the heterodimer of SSTR1 and SSTR4; and (11) the method according to any one of (6) to (8) above, which is a method for screening a compound for treating and/or preventing a disease caused by accumulation of Aβ protein.

### Effect of the Invention

According to the present invention, it is possible to screen a compound that can interact with a heterodimer of SSTR1 and SSTR4 to induce activation of neprilysin. Such a compound can be utilized as a therapeutic and/or preventive agent for diseases caused by accumulation of Aβ since it is likely to accelerate degradation of Aβ through the activation of neprilysin.

### Brief Description of Drawings

[Figure 1] Figure 1 is a diagram illustrating experimental procedures in the following Examples.
[Figure 2] Figure 2 is a diagram illustrating results of investigation by immunostaining into the expression of SSTR1 protein and SSTR4 protein in CHO cells into which an SSTR1 gene and an SSTR4 gene have been introduced.
[Figure 3] Figure 3 is a diagram illustrating results of investigation by proximity ligation assay into interaction of SSTR1 protein with SSTR4 protein in CHO cells into which an SSTR1 gene and an SSTR4 gene have been introduced.

### Mode of Carrying Out the Invention

The transformant of the present invention is not particularly restricted as long as it is a transformant expressing a heterodimer of SSTR1 protein and SSTR 4 protein into which a DNA encoding the SSTR1 protein and a DNA encoding the SSTR4 protein are introduced (hereinafter, "DNA encoding the SSTR1 protein" and "DNA encoding the SSTR4 protein" may be respectively referred to as an "SSTR1 gene" and an "SSTR4 gene," and these two DNAs may be collectively referred to as an "SSTR1/4 gene." Hereinafter, the "heterodimer of the SSTR1 protein and the SSTR4 protein" may also be referred to as an "SSTR1/4 heterodimer"). In the present invention, the "SSTR1 protein" and the "SSTR4 protein" each mean a known G protein-coupled receptor described in Olias et al.(Journal of Neurochemistry, 2004, 89, 1057-1091), Moller et al. (Biochimica et Biophysica Acta, 2003, 1616, 1-84), and the like, while in the present invention, the "SSTR1/4 heterodimer" means a dimer formed by association of these two different subtypes of receptor proteins. The transformant of the present invention is also characterized in that the SSTR1 protein and the SSTR4 protein derived from a foreign SSTR1/4 gene introduced are expressed, and the SSTR1/4 heterodimer containing them is expressed in the cell or on the cell surface.

The "SSTR1 gene" may be any DNA as long as it contains a nucleotide sequence encoding the SSTR1 protein, and the "SSTR4 gene" may be any DNA as long as it contains a nucleotide sequence encoding the SSTR4 protein, but both of these genes are preferably derived from mammals such as humans, mice, rats, rabbits, cattle, and pigs and further preferably derived from humans. Specific examples of the "SSTR1 gene" and the "SSTR4 gene" suitably include a nucleotide sequence shown in positions 1 to 1173 of SEQ ID No: 1 and a nucleotide sequence shown in positions 1 to 1164 of SEQ ID No: 2, respectively. In addition, the SSTR1/4 gene may further contain a nucleotide sequence encoding a marker protein and/or a peptide tag for purification, detection, and quantification of the protein. The "marker protein" is not particularly limited, and examples thereof suitably include a conventionally known marker protein such as alkaline phosphatase, a Fc region of an antibody, HRP, and GFP, while examples of the "peptide tag" suitably include a conventionally known peptide tag such as Myc tag, a FLAG tag, His tag, and GST tag.

The transformant of the present invention can be prepared by introducing the SSTR1/4 gene into a host cell. The SSTR1/4 gene may be integrated into a vector that can be expressed in the host cell, both genes may be integrated into one vector, and each may be integrated into a separate vector. Examples of such a "vector" suitably include an expression system derived from chromosomes, episomes, and viruses, such as a vector derived from bacterial plasmid; a vector derived from yeast plasmid; a vector derived from a papovavirus like SV40, vaccinia virus, adenovirus, fowlpox virus, pseudorabies virus, and retrovirus; a vector derived from bacteriophage or transposon; and a vector derived from a combination thereof, such as a vector derived from genetic factors of plasmid and bacteriophage like cosmid and phagemid. These vectors may also contain a control sequence that not only causes expression but also regulates expression. Specific examples thereof suitably include a known plasmid such as pcDNA3.1, pcDNA6.2, pUC18, p3XFLAG-CMV10, and AcNPV.

The "host cell" may be any cell as long as it can express the SSTR1/4 heterodimer, and examples thereof suitably include a bacterial prokaryotic cell such as *E. coli, Streptomyces, Bacillus Subtilis, Streptococcus,* and *Staphylococcus;* a fungal cell such as yeast and *Aspergillus;* a cell line derived from insects such as *Drosophila S2* and *Spodoptera Sf9;* and a cell line derived from mammals such as CHO cell, N1E-115 cell, SH-SY5Y cell, HeLa cell, L cell, COS cell, C127 cell, BHK21 cell, HEK293 cell, and Bowes melanoma cell, and among them, a CHO cell is preferred. The "host cell" may also be a primary cultured nerve cell collected from an insect or a mammal. Such a "primary cultured nerve cell" can be collected from any of brain regions (for example, hippocampus, thalamus, hypothalamus, subthalamic nucleus, cerebral cortex, medulla oblongata, cerebellum, occipital lobe, frontal lobe, temporal lobe, putamen, caudate nucleus, corpus callosum, substantia nigra, olfactory bulb, amygdaloid nucleus, and basal ganglia) using a known method (such as a method described in Culturing Nerve Cells (The MIT press, 1991)) and the like), and among them, a primary cultured nerve cell collected from the hippocampus or cerebral cortex is preferred.

Introduction of the gene into the host cell can be performed through methods described in many standard laboratory manuals such as Davis et al. (BASIC METHODS IN MOLECULAR BIOLOGY, 1986) and Sambrook et al. (MOLECULAR CLONING: A LABORATORY MANUAL, 2nd Ed., Cold Spring Harbor Laboratory Press, Cold Spring Harbor, N.Y., 1989), for example, lipofection, calcium phosphate transfection, DEAE-dextran mediated transfection, transvection, microinjection, cationic lipid-mediated transfection, electroporation, transduction, scrape loading, ballistic introduction, and infection.

In addition, a method for confirming the expression of SSTR1 protein and SSTR4 protein in the host cell after the introduction of the gene is not particularly restricted as long as it is a known protein engineering method, and examples thereof include Immunostaining, Western blotting, immunoprecipitation, and ELISA using an antibody that specifically recognizes the SSTR1 protein and/or the SSTR4 protein or an antibody that specifically recognizes a marker protein or a peptide tag contained in the SSTR1/4 gene. Also, a method for confirming the formation of an SSTR1/4 heterodimer on the cell surface or in the cell of the transformant of the present invention is not particularly limited as long as it is a known protein engineering method, and specific examples thereof suitably include proximity ligation assay method, fluorescence resonance energy transfer method (FRET), immunohistochemistry, Western blotting, immunoprecipitation, bioluminescence resonance energy transfer (BRET), affinity chromatography, biomolecular fluorescence complementation, and scintillation proximity assay (SPA), but among them, particularly suitably include a confirmation method of expressing SSTR1 protein and SSTR4 protein which have been each differently labeled in the transformant of the present invention, and determining the proximity between the different labels by a proximity ligation assay method. The proximity ligation assay method can be performed using a commercially available product such as Duolink (R) (manufactured by Sigma-Aldrich), and a signal obtained through the proximity ligation assay method can be detected and analyzed using standard fluorescence microscopy and image analysis software programs.

The transformant of the present invention can be used to elucidate a signal transduction system involving the SSTR1/4 heterodimer. The "signal transduction system" may be any intracellular signal transduction system as long as the system is specifically activated via the SSTR1/4 heterodimer, but preferably it is an intracellular signal transduction system involved in the activation of neprilysin. The "neprilysin" is one type of known neutral endopeptidase described in Saido (Aβ METABOLISM AND ALZHEIMER'S DISEASE, 2003, ISBN: 1-58706-230-5) and the like, which is present in various tissues of animals and is known to have Aβ-degrading activity. In other words, the transformant of the present invention may be limited to the transformant for use in application of "elucidation of the signal transduction system involved in the activity of neprilysin via the SSTR1/4 heterodimer." The transformant of the present invention for use in such application may also be one into which a DNA encoding neprilysin is further introduced in addition to the SSTR1/4 gene. The "DNA encoding neprilysin" may be any DNA as long as it contains a nucleotide sequence encoding a neprilysin protein, but is preferably derived from mammals such as humans, mice, rats, rabbits, cattle, and pigs (hereinafter, the "DNA encoding neprilysin" is referred to as the "neprilysin gene"). The introduction of the neprilysin gene can be performed in the same manner as the method for introducing the SSTR1/4 gene into a host cell.

Examples of a method for "elucidating the signal transduction system involved in the activity of neprilysin via the SSTR1/4 heterodimer" using the transformant of the present invention include a method for measuring the activity of neprilysin according to known methods described in Japanese unexamined Patent Application Publication No. 2002-34596, Japanese unexamined Patent Application Publication No. 2004-151079, and the like, comprising incubating a compound that can interact with the SSTR1/4 heterodimer (such as somatostatin, a known somatostatin analog, and any test compound) with the transformant of the present invention and then either fixing such a transformant or preparing a cell extract or a neprilysin extract from such a transformant.

Specifically, the "activity of neprilysin" can be calculated by using a substrate for neprilysin to measure the amount of the substrate degraded. The "substrate for neprilysin" is not particularly limited, and examples thereof suitably include a synthetic compound such as benzyloxycarbonyl-alanyl-alanyl-leucyl-paranitroanilide, benzyloxycarbonyl-alanyl-alanyl-phenylalanyl-paranitroanilide, benzyloxycarbonyl-glycyl-glycyl-leucyl-paranitroanilide, benzyloxycarbonyl-glycyl-glycyl-phenylalanyl-paranitroanilide, glutaryl-alanyl-alanyl-phenylalanyl-4-methoxy-2-naphthylamide, glutaryl-alanyl-alanyl-phenylalanyl-2-naphthylamide, and succinyl-alanyl-alanyl-phenylalanine-4-methylcoumarin-7-amide; and a peptide such as an enkephalin, substance P, atrial natriuretic peptide (ANP), gastrin-releasing peptide (GRP), and endothelin. In addition, conditions of the "incubation" can be appropriately selected by those skilled in the art depending on the substrate to be used. For example, the concentration of the substrate varies depending on the substrate used, but a reaction may be carried out at 0.1 to 1000 µg/ml. The concentration of the substrate is preferably 1 to 100 µg/ml and further preferably 3 to 30 µg/ml in the reaction system. The reaction temperature is preferably 20°C to 45°C and further preferably 20°C to 40°C. The reaction time varies depending on conditions of a reaction system such as a substrate to be used and its concentration, but for example, it may be appropriately selected from 5 minutes to 24 hours. In terms of rapidity, it is preferable to set the reaction system such that measurements can be made in a short time of 5 to 60 minutes. The reaction is preferably carried out at a neutral pH, that is, pH 6 to 9, and more preferably at pH 7 to 8.

Furthermore, the "amount of degraded neprilysin substrate" can be calculated by measuring concentration of a compound generated by degradation (hereinafter may be referred to as a "degradation product"). Specifically, for example, if the degradation product emits fluorescence or if the degradation product reacts with a reagent to emit fluorescence, the amount of the degraded neprilysin substrate can be measured by measuring its fluorescence intensity. As another aspect, an amount of the degradation product and/or neprilysin substrate can also be analyzed by thin-layer chromatography, HPLC, or the like. At this point, the amount of degradation may be corrected with a value measured by adding an inhibitor (for example, thiorphan). The activity of neprilysin may also be taken as the amount of substrate degraded per unit cell mass. The "activity of neprilysin" data thus obtained can be used to elucidate an intracellular signal transduction system that activates neprilysin via the SSTR1/4 heterodimer.

Furthermore, the transformant of the present invention can also be used to prepare the "isolated SSTR1/4 heterodimer" of the present invention. For example, an SSTR1/4 heterodimer can be recovered and purified from a culture in which the transformant of the present invention is cultured, using known methods such as ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography to obtain the "isolated SSTR1/4 heterodimer." In addition, as columns used for the affinity chromatography, it is possible to use, for example, a column to which an anti-SSTRl monoclonal antibody or an anti-SSTR4 monoclonal antibody is bound, and a column to which substances having affinity to a marker protein or a peptide tag attached to the SSTR1/4 heterodimer are bound.

The screening method of the present invention is not particularly limited as long as it comprises steps (a), (b), and (c): (a) a step of bringing a test compound into contact with an SSTR1/4 heterodimer; (b) a step of measuring interaction of the heterodimer with the test compound; and (c) a step of selecting a compound that interacts with the heterodimer based on the measurement results in (b) above (hereinafter, the above steps (a) to (c) may be referred to as the "first step"). The screening method of the present invention may further comprise steps (d'), (e), and (f): (d') a step of bringing the test compound into contact with a homodimer of SSTR1 and/or a homodimer of SSTR4; (e) a step of measuring the interaction of the test compound with the homodimer; and (f) a step of selecting a compound having a weaker interaction than the interaction measured in (b) above based on the measurement results in (e)above (hereinafter, the above steps (d') to (f) may be referred to as the "second step"). The first step and the second step may be carried out in the order of the first step followed by the second step, or may be carried out in the order of the second step followed by the first step, or may be carried out simultaneously, but it is preferable to carry out in the order of the first step followed by the second step. In other words, it is more preferable for the screening method of the present invention to comprise the first step followed by steps (d), (e), and (f): (d) a step of bringing the test compound selected in (c) above into contact with a homodimer of SSTR1 and/or a homodimer of SSTR4; (e) a step of measuring the interaction of the test compound with the homodimer; and (f) a step of selecting a compound having a weaker interaction than the interaction measured in (b) of the first step based on the measurement results in (e) above.

The "SSTR1/4 heterodimer" used in the step (a) of the screening method of the present invention may be an SSTR1/4 heterodimer expressed on a cell surface or an isolated one. For example, in the above step (a), as the "SSTR1/4 heterodimer," the transformant of the present invention may be used as it is, or a cell extract or a membrane fraction containing the "SSTR1/4 heterodimer" derived from such a transformant may be used, and the isolated SSTR1/4 heterodimer of the present invention may be used. Similarly, the "homodimer of SSTR1" and the "homodimer of SSTR4" used in the above step (d') or (d) may be a homodimer of SSTR1 or SSTR4 expressed on the cell surface or an isolated one. For example, a transformant into which each of the SSTR1 gene and the SSTR4 gene has been introduced independently may be prepared by the same method as for preparing the transformant of the present invention, and as the "homodimer of SSTR1" or the "homodimer of SSTR4," such a transformant may be used as it is, or a cell extract or a membrane fraction containing a homodimer of SSTR1 or SSTR4 derived from such a transformant may be used. Furthermore, the "isolated SSTR1 homodimer or SSTR4 homodimer" can be prepared by the same method as for preparing the isolated SSTR1/4 heterodimer of the present invention and used as the "homodimer of SSTR1" or the "homodimer of SSTR4."

The "test compound" in the screening method is not particularly limited as long as it is a compound that can bind to the SSTR1/4 heterodimer, and may be a known compound or a novel compound. Examples of the "test compound" include a protein (including an antibody), a peptide, a non-peptide compound (such as nucleotide, amine, sugar, and lipid), an organic low-molecular-weight compound, an inorganic low-molecular-weight compound, a fermentation product, a cell extract, a plant extract, and an animal tissue extract, and also suitably include a known compound library and peptide library such as a natural compound library, an allosteric compound library, a peptide library, an antibody fragment library, a synthetic compound library, a fragment-based library, and a phage display library. The "test compound" may also be a known peptide analog of somatostatin (for example, U.S. Patent Nos. 4,485,101 and 5,409,894; or International Publication No. WO 97/47317) and a known non-peptide SSTR agonist (International Publication No. WO 97/03054; U.S. Patent No. 6,221,870; or U.S. Patent Nos. 6,329,389 and 6,352,982). The form of the test compound is not particularly limited, and examples thereof include a solid, liquid, mixture with a base agent, a suspension, or a solution. In the case of a suspension or a solution, water, pH buffer, methylcellulose solution, a saline solution, an aqueous organic solvent, or the like can be used.

In the above steps (a), (d'), and (d), the SSTR1/4 heterodimer can be brought into contact with the test compound by a usual method. The amount, concentration, number of contacts, and contact time of the test compound used in such steps can be selected appropriately by those skilled in the art within a range that does not seriously affect the structure of the SSTR1/4 heterodimer. For example, if the transformant is used as it is in the above step (a), (d'), or (d), the test compound is added to a culture solution and agar culture medium containing such a transformant or the like at a concentration of about 0.001 to 100 µM, thereby bringing the SSTR1/4 heterodimer into contact with the test compound.

In the above steps (b) and (e), methods common in the art such as ELISA, surface plasmon resonance, and phage display can be used as methods for measuring the interaction of test compound with the heterodimer or the homodimer (for example, Sambrook et al. Molecular Cloning, A Laboratory Manual, Third Ed., Cold Spring Harbor Laboratory Press (2001)). The presence or absence of the interaction or the intensity of the interaction of the test compound with the heterodimer or the homodimer are each measured by these methods, and based on the results, the test compound which is found to interact with the heterodimer can be selected as a "compound that interacts with the SSTR1/4 heterodimer" in the above step (c). In the above step (f), the test compound having a weaker intensity of interaction with the homodimer than that of interaction with the heterodimer can be selected as a "compound that interacts with the SSTR1/4 heterodimer" by comparing the measurement results in step (b) above with those in step (e) above. In other words, a compound that specifically binds to the SSTR1/4 heterodimer but not to (or only exhibits a low affinity to) the homodimer of SSTR1 or the homodimer of SSTR4 can be selected through the above step (f).

In addition, the screening method of the present invention may further comprise (a') a step of selecting a test compound that has the possibility to interact with the SSTR1/4 heterodimer by in silico screening. Here, it is preferable to carry out step (a') before step (a) from the viewpoint of efficiency. The in silico screening can be performed by any method known to those in the art using known docking software such as DOCK, FlexX, AutoDock, GOLD, Glide, and Ph4Dock based on the three-dimensional structure information of the SSTR1/4 heterodimer obtained by X-ray structural analysis, cryomicroscopic analysis, or the like. The above step (a') may also be a step of selecting a test compound using a known compound database such as ChEMBL (http//www.ebi.ac.uk/chembl/), DrugBank (http//www.drugbank.ca/), CTD (http://ctd.mdibl.org/), and PubChem BioAssay (http://www.ncbi.nlm.nih.gov/pcassay).

The "compound" selected by the screening method of the present invention is preferably an agonist or allosteric modulator of the SSTR1/4 heterodimer. Here, the "agonist" means a ligand that binds to an orthosteric site of a receptor and increases the signal transduction activity of the receptor and includes a full agonist capable of maximum receptor stimulation and a partial agonist unable to elicit full activity even at saturating concentrations. The "compound" in the present invention may be either a full agonist or a partial agonist but is preferably a partial agonist. In addition, the "allosteric modulator" means a substance that binds to an allosteric site of a G protein-coupled receptor (in other words, a regulatory site physically distinct from an active site of the protein) and regulates signal transduction activity of the receptor. In contrast to an orthosteric ligand, an allosteric modulator, even if an endogenous ligand is also bound, is known to modify receptor function by binding to the receptor at a different site and altering the affinity of such an endogenous ligand for the receptor, thereby controlling the strength of activation or deactivation of the receptor. Although allosteric modulators are known to include a positive allosteric modulator that increases the activity of a receptor and a negative allosteric modulator that suppresses the activity of a receptor, the "compound" in the present invention is preferably a positive allosteric modulator.

The "compound" can be used to identify an organ, tissue, or cell expressing an SSTR1/4 heterodimer. For example, the compound may be labeled with a radioisotope such as ⁶⁷Ga, ⁶⁸Ga, ⁹⁹mTc (Tc-⁹⁹m) , ¹¹¹In, ¹²³I, and ¹²⁵I and administered to humans or non-human animals followed by scintigraphy, thereby identifying tissues and cells expressing the SSTR1/4 heterodimer *in vivo.* In addition, when the "compound" is an antibody, a labeled antibody is prepared with a fluorescent substance such as FITC (fluorescein isocyanate) or tetramethylrhodamine isothiocyanate, a radioisotope such as ¹²⁵I, ³²P, ¹⁴C, ³⁵S, and ³H, and an enzyme such as alkaline phosphatase, peroxidase, β-galactosidase, or phycoerythrin, and the expression of the SSTR1/4 heterodimer in tissues and cells collected from living organisms can be identified by methods such as RIA, ELISA, a fluorescent antibody method, a plaque method, a spot method, a hemagglutination method, and an Ouchterlony method.

The "compound" selected by the screening method of the present invention has the possibility to activate neprilysin via an SSTR1/4 heterodimer and accelerate degradation of Aβ levels. Accordingly, the screening method of the present invention can be employed as the method for screening a compound that improves the activity of neprilysin, and the screening method of the present invention can also be employed as the method for screening a compound for treating and/or preventing a disease caused by the accumulation of Aβ protein. Specifically, (1) after identifying an agonist or allosteric agonist specific to the SSTR1/4 heterodimer by the screening method of the present invention, (2) such an agonist or allosteric agonist was chemically modified to improve specificity and permeability of the blood-brain barrier, and (3) Aβ accumulation suppressing effect and safety were confirmed using a next-generation mouse model of Alzheimer's disease (Saito et al., Nat Neurosci 17(5): 661-663, 2014. Doi: 10.1038/nn.3697.), thereby enabling (4) a search for therapeutic and/or preventive agent for a disease caused by the accumulation of Aβ protein, which can be applied in clinical trials. In the present invention, the "disease caused by the accumulation of Aβ protein" means a disease or condition in which a causal relationship between Aβ levels and the disease is suggested *in vivo* and includes Alzheimer's disease, cerebrovascular amyloid angiopathy, cerebrovascular dementia, other dementia, cerebral infarction, and the like, in which accumulation of Aβ in a brain parenchyma region (called senile plaques) or the accumulation in the cerebral blood vessels is observed. The compound is expected to have a preventive effect on patients before the onset of the "disease caused by the accumulation of Aβ protein" (effect of suppressing an increase in Aβ protein levels) and a therapeutic effect on patients who are diagnosed to have the "disease caused by the accumulation of Aβ protein" (effect of improving symptoms and delaying progression by degradation of already accumulated Aβ protein). The transformant of the present invention can be used for the application of the method for screening a compound that improves the activity of neprilysin and also used for the application of the method for screening a compound for treating and/or preventing a disease caused by accumulation of Aβ protein.

### Examples

Hereinafter, the present invention will be described in more detail by referring to the Examples, while the technical scope of the present invention is not limited to these exemplifications.

### Example 1

### (1) Preparation of SSTR1- and SSTR4-expressing CHO Cells (1-1) Culture of CHO Cells

A 24-well plate for cell culture (manufactured by Thermo Fisher Scientific) with a coverslip (manufactured by Matsunami Glass Ind., Ltd) placed therein was coated with poly-L-lysine (PLL; manufactured by Sigma) to prepare a culture plate. Next, CHO cells were suspended in a culture solution (DMEM containing 10% FBS (manufactured by Nichirei Bioscience), penicillin, and streptomycin (manufactured by NACALAI TESQUE, INC.); manufactured by Gibco) and seeded at 2.7 × 10⁴/500 µL/well in two of the culture plates. The two plates were provided each for immunostaining and proximity ligation assay.

### (1-2) Preparation of Mixed solution for Transfection

The following DNA sequence encoding human SSTR1 (SEQ ID No: 1) or DNA sequence encoding human SSTR4 (SEQ ID No: 2) was inserted into pcDNA (manufactured by Thermo Fisher Scientific) to prepare expression vectors for human SSTR1 and human SSTR4 (hereinafter, such expression vectors are each described as "pcDNA-SSTR1" and "pcDNA-SSTR4").

Human SSTR1 (wherein the underlined part indicates the Flag-tag sequence):

ATGTTCCCCAATGGCACCGCCTCCTCTCCTTCCTCCTCTCCTAGCCCCAGCCCGGGCA

GCTGCGGCGAAGGCGGCGGCAGCAGGGCCCCGGGGCCGGCGCTGCGGACGGCATGGA

GGAGCCAGGGCGAAATGCGTCCCAGAACGGGACCTTGAGCGAGGGCCAGGGCAGCGCC

ATCCTGATCTCTTTCATCTACTCCGTGGTGTGCCTGGTGGGGCTGTGTGGGAACTCTA

TGGTCATCTACGTGATCCTGCGCTATGCCAAGATGAAGACGGCCACCAACATCTACAT

CCTAAATCTGGCCATTGCTGATGAGCTGCTCATGCTCAGCGTGCCCTTCCTAGTCACC

TCCACGTTGTTGCGCCACTGGCCCTTCGGTGCGCTGCTCTGCCGCCTCGTGCTCAGCG

TGGACGCGGTCAACATGTTCACCAGCATCTACTGTCTGACTGTGCTCAGCGTGGACCG

CTACGTGGCCGTGGTGCATCCCATCAAGGCGGCCCGCTACCGCCGGCCCACCGTGGCC

AAGGTAGTAAACCTGGGCGTGTGGGTGCTATCGCTGCTCGTCATCCTGCCCATCGTGG

TCTTCTCTCGCACCGCGGCCAACAGCGACGGCACGGTGGCTTGCAACATGCTCATGCC

AGAGCCCGCTCAACGCTGGCTGGTGGGCTTCGTGTTGTACACATTTCTCATGGGCTTC

CTGCTGCCCGTGGGGGCTATCTGCCTGTGCTACGTGCTCATCATTGCTAAGATGCGCA

TGGTGGCCCTCAAGGCCGGCTGGCAGCAGCGCAAGCGCTCGGAGCGCAAGATCACCTT
AATGGTGATGATGGTGGTGATGGTGTTTGTCATCTGCTGGATGCCTTTCTACGTGGTG
CAGCTGGTCAACGTGTTTGCTGAGCAGGACGACGCCACGGTGAGTCAGCTGTCGGTCA
TCCTCGGCTATGCCAACAGCTGCGCCAACCCCATCCTCTATGGCTTTCTCTCAGACAA
CTTCAAGCGCTCTTTCCAACGCATCCTATGCCTCAGCTGGATGGACAACGCCGCGGAG
GAGCCGGTTGACTATTACGCCACCGCGCTCAAGAGCCGTGCCTACAGTGTGGAAGACT
TCCAACCTGAGAACCTGGAGTCCGGCGGCGTCTTCCGTAATGGCACCTGCACGTCCCG
GATCACGACGCTCGATTACAAGGACGACGACGATAAGTGA.

Human SSTR4 (wherein the underlined part indicates the Myc-tag sequence):

ATGAGCGCCCCCTCGACGCTGCCCCCCGGGGGCGAGGAAGGGCTGGGGACGGCCTGGC
CCTCTGCAGCCAATGCCAGTAGCGCTCCGGCGGAGGCGGAGGAGGCGGTGGCGGGGCC
CGGGGACGCGGGCGGCGGGCATGGTCGCTATCCAGTGCATCTACGCGCTGGTGTGC
CTGGTGGGGCTGGTGGGCAACGCCCTGGTCATCTTCGTGATCCTTCGCTACGCCAAGA
TGAAGACGGCTACCAACATCTACCTGCTCAACCTGGCCGTAGCCGACGAGCTCTTCAT
GCTGAGCGTGCCCTTCGTGGCCTCGTCGGCCGCCCTGCGCCACTGGCCCTTCGGCTCC
GTGCTGTGCCGCGCGGTGCTCAGCGTCGACGGCCTCAACATGTTCACCAGCGTCTTCT
GTCTCACCGTGCTCAGCGTGGACCGCTACGTGGCCGTGGTGCACCCTCTGCGCGCGGC
GACCTACCGGCGGCCCAGCGTGGCCAAGCTCATCAACCTGGGCGTGTGGCTGGCATCC
CTGTTGGTCACTCTCCCCATCGCCATCTTCGCAGACACCAGACCGGCTCGCGGCGGCC
AGGCCGTGGCCTGCAACCTGCAGTGGCCACACCCGGCCTGGTCGGCAGTCTTCGTGGT
CTACACTTTCCTGCTGGGCTTCCTGCTGCCCGTGCTGGCCATTGGCCTGTGCTACCTG
CTCATCGTGGGCAAGATGCGCGCCGTGGCCCTGCGCGCTGGCTGGCAGCAGCGCAGGC
GCTCGGAGAAGAAAATCACCAGGCTGGTGCTGATGGTCGTGGTCGTCTTTGTGCTCTG
CTGGATGCCTTTCTACGTGGTGCAGCTGCTGAACCTCTTCGTGACCAGCCTTGATGCC
ACCGTCAACCACGTGTCCCTTATCCTTAGCTATGCCAACAGCTGCGCCAACCCCATTC
TCTATGGCTTCCTCTCCGACAACTTCCGCCGATTCTTCCAGCGGGTTCTCTGCCTGCG
CTGCTGCCTCCTGGAAGGTGCTGGAGGTGCTGAGGAGGAGCCCCTGGACTACTATGCC
ACTGCTCTCAAGAGCAAAGGTGGGGCAGGGTGCATGTGCCCCCCACTCCCCTGCCAGC

AGGAAGCCCTGCAACCAGAACCCGGCCGCAAGCGCATCCCCCTCACCAGGACCACCAC
CTTCGAACAAAAACTAATATCAGAAGAAGACCTATGA.

Next, a plasmid mixture was prepared so that pcDNA-SSTR1 and pcDNA-SSTR4 each had a final concentration of 1 mg/ml. Then, Opti-MEM (manufactured by Gibco) and the plasmid mixture were added to a tube and stirred with a vortex mixer, and FuGENE-HD (manufactured by Promega) was further added and mixed by pipetting 15 times or vortex mixer to prepare a mixed solution for transfection. The amount of each solution used here is as follows:
plasmid mixture: 17 µL
Opti-MEM: 1,034 µL
FuGENE-HD: 66 µL.

### (1-3) Transfection into CHO Cell

The mixed solution for transfection prepared in (1-2) above was incubated at room temperature for 5 to 10 minutes, then added to the two plates prepared in (1-1) above (25 µL/well), mixed slowly, and cultured for another 24 hours. In each plate, 20 wells were transfection treated, while 4 wells were untreated to make control groups.

### Example 2

### (2) Cell Fixation and Permeabilization

### (2-1) Cell Fixation

After removal of the culture solution from the cells obtained in (1-3) above, a phosphate buffer containing 3.7% paraformaldehyde (PFA-phosphate buffer) was added (300 µL/well), and the cells were incubated at room temperature for 15 minutes for fixation. After incubation, the PFA-phosphate butter was removed, and then an operation of adding PBS and incubating for 10 minutes was repeated three times to wash the cells.

### (2-2) Cell Permeabilization

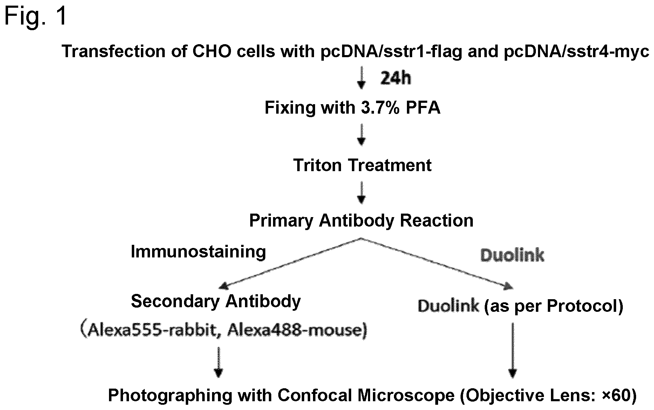

Next, PBS containing 0.1% or 0.5% TritonX100 (manufactured by NACALAI TESQUE, INC) (Triton-PBS) was added to the cells obtained in (2-1) above (25 µL/well) and incubated at room temperature for 5 minutes for permeabilization. After incubation, Triton-PBS was removed, and then an operation of adding PBS and incubating for 10 minutes was repeated three times to wash the cells. As illustrated in Figure 1, these cells were subjected to immunostaining (Example 3) and proximity ligation assay (Example 4). In these experiments, four experimental groups were set up as shown in Table 1 below, in which different concentrations of TritonX100-PBS and combinations of the primary antibodies were used for each.

**[Table 1]**

| | Experimental group A | Experimental group B | Experimental group C | Experimental group D |
|---|---|---|---|---|
| Triton (permeabilization) | 0.1% | 0.1% | 0.5% | 0.5% |
| Anti-FLAG antibody (primary antibody) | 1000-fold dilution | 200-fold dilution | 1000-fold dilution | 200-fold dilution |
| Anti-Myc antibody (primary antibody) | 8000-fold dilution | 1000-fold dilution | 8000-fold dilution | 1000-fold dilution |

### Example 3

### (3) Immunostaining

### (3-1) Blocking

PBS containing 10% normal goat serum (10% NGS-PBS) was added to the cells obtained in (2-2) above and incubated at room temperature for 30 minutes for blocking treatment. After incubation, 10% NGS-PBS was removed, and then PBS was added and incubated for 5 minutes to wash the cells.

### (3-2) Primary Antibody

An anti-FLAG antibody (DYKDDDDK Tag (D6W5B) rabbit monoclonal antibody; CST #14793; manufactured by Cell Signaling Technology, Inc.), and anti-Myc antibody (Myc-Tag (9B11) mouse monoclonal antibody; CST #2276; manufactured by Cell Signaling Technology, Inc.) were diluted using PBS containing 5% normal goat serum (5% NGS-PBS) to prepare a primary antibody mixture for immunostaining. The dilution ratio of each antibody is as shown in Table 1. The primary antibody mixture for immunostaining was added to the cells obtained in (3-1) above and incubated at 4°C overnight. After incubation, the primary antibody mixture for immunostaining was removed, and an operation of adding PBS and incubating for 5 minutes was repeated three times to wash the cells.

### (3-3) Secondary Antibody

An Alexa Fluor Plus 555-labeled anti-rabbit antibody (manufactured by Thermo Fisher Scientific) and an Alexa Fluor Plus 488-labeled anti-mouse antibody (manufactured by Thermo Fisher Scientific) were diluted 1000-fold with PBS to prepare a secondary antibody mixture for immunostaining. The secondary antibody mixture for immunostaining was added to the cells obtained in (3-2) above and incubated at room temperature for 1 hour. After incubation, the secondary antibody mixture for immunostaining was removed, and then an operation of adding PBS and incubating for 10 minutes was repeated three times to wash the cells.

### (3-4) Embedding

The cells stained in (3-3) above were embedded into a Prolong Gold antifade reagent (manufactured by Thermo Fisher Scientific) and stored at 4°C for 1 day until observation.

### (3-5) Observation

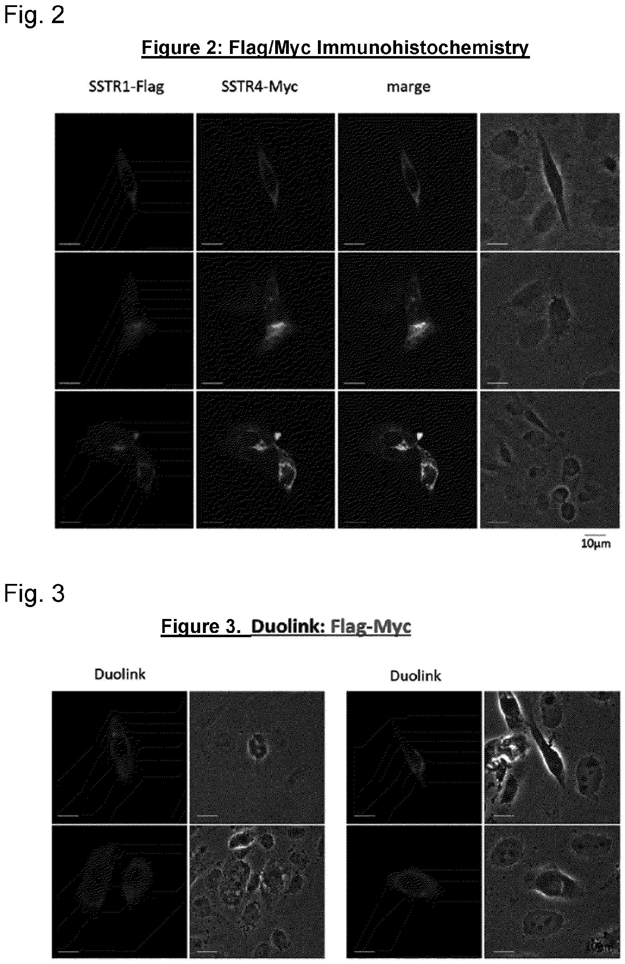

As illustrated in Figure 2, as a result of observation with a confocal microscope (FLUOVIEW Fv10i; manufactured by Olympus Corporation), it was revealed that SSTR1 and SSTR4 proteins were co-expressed in the cells prepared in Example 1. In addition, the localization of the SSTR1 and SSTR4 proteins was consistent, suggesting that the SSTR1 and SSTR4 proteins may form a heterodimer on the cell membrane.

### Example 4

### (4) Proximity Ligation Assay

### (4-1) Blocking

A proximity ligation assay was performed using a Duolink PLA kit (manufactured by Sigma-Aldrich). First, a Duolink blocking solution was added to the cells obtained in (2-2) above and incubated at 37°C for 60 minutes for blocking treatment. After incubation, the Duolink blocking solution was removed, and then PBS was added and incubated for 5 minutes to wash the cells.

### (4-2) Primary Antibody

The anti-FLAG antibody and anti-Myc antibody were diluted with the Duolink antibody diluent as shown in Table 1 to prepare a primary antibody mixture for Duolink. The primary antibody mixture for Duolink was added to the cells obtained in (4-1) above and incubated at 4°C overnight. After incubation, the primary antibody mixture for Duolink was removed, and then an operation of adding PBS and incubating for 5 minutes was repeated twice to wash the cells.

### (4-3) PLA Probe

For the preparation of 50 µL of PLA probe solution, 10 µL of PLA probe anti-rabbit (+), 10 µL of PLA probe anti-mouse (-), and 30 µL of PBS were mixed. The PLA probe solution was added to the cells obtained in (4-2) above and incubated in a constant humidity chamber at 37°C for 1 hour. After incubation, the PLA probe solution was removed, and then an operation of adding a wash buffer A (0.01 M Tris, 0.15 M NaCl, and 0.05% Tween 20) and incubating for 5 minutes was repeated twice to wash the cells.

### (4-4) Ligation

For the preparation of 50 µL of ligase solution, 10 µL of 5X Ligation Stock (manufactured by Sigma), 1.25 µL of DNA Ligase (manufactured by Sigma), and 38.75 µL of water were mixed. The ligase solution was added to the cells obtained in (4-3) above and incubated in a constant humidity chamber at 37°C for 30 minutes. After incubation, the ligase solution was removed, and then an operation of adding the wash buffer A and incubating for 5 minutes was repeated twice to wash the cells.

### (4-5) Elongation

For the preparation of 50 µL of amplification solution, 10 µL of 5X Amplification Buffer (manufactured by Sigma), 0.625 µL of DNA polymerase (manufactured by Sigma), and 39.4 µL of water were mixed. The amplification solution was added to the cells obtained in (4-4) above and incubated in a constant humidity chamber at 37°C for 100 minutes. After incubation, the amplification solution was removed, and then an operation of adding a wash buffer B (0.2 M Tris and 0.1 M NaCl) and incubating for 10 minutes was repeated twice to wash the cells.

### (4-6) Embedding

The cells obtained in (4-4) above were embedded into a Prolong Gold Antifade Reagent (manufactured by Thermo Fisher Scientific) and stored at 4°C for 1 day until detection.

### (4-7) Detection

As illustrated in Figure 3, as a result of imaging with a confocal microscope (FLUOVIEW FV10i; manufactured by Olympus Corporation), a fluorescent spot of PLA signal was confirmed in the cell prepared in Example 1. This result supports the interaction of the SSTR1 protein with the SSTR4 protein. From the results of Examples 3 and 4 above, it was confirmed that the SSTR1 and SSTR4 proteins form a heterodimer on the cell membrane.

### Industrial Applicability

According to the present invention, it is possible to demonstrate the existence of a heterodimer of SSTR1 and SSTR4, and also to provide a method for screening a factor that binds to such a heterodimer and the heterodimer that can be used in the screening method, thus enabling a search for a preventive and/or therapeutic agent for a disease caused by Aβ deposition such as Alzheimer's disease. The transformant of the present invention can be used in the preparation of an isolated SSTR1/4 heterodimer, elucidation of a signal transduction system involving a heterodimer of somatostatin receptor subtype 1 and somatostatin receptor subtype 4, a method for screening a compound that improves the activity of neprilysin, a method for screening a compound for treating and/or preventing a disease caused by accumulation of Aβ protein, and the like. The screening method of the present invention can be employed as the method for screening a compound that improves the activity of neprilysin and also employed as the method for screening a compound for treating and/or preventing a disease caused by the accumulation of Aβ protein. It is also possible to screen a compound that can interact with a heterodimer of SSTR1 and SSTR4 to induce activation of neprilysin, and such a compound can be utilized as a therapeutic and/or preventive agent for a disease caused by the accumulation of Aβ because it is likely to accelerate degradation of Aβ through the activation of neprilysin.

## Claims

1. A transformant expressing a heterodimer of somatostatin receptor subtype 1 and somatostatin receptor subtype 4, into which a DNA encoding somatostatin receptor subtype 1 and a DNA encoding somatostatin receptor subtype 4 have been introduced.

2. The transformant according to claim 1, wherein a vector comprising the DNA encoding somatostatin receptor subtype 1 and a vector comprising the DNA encoding somatostatin receptor subtype 4 have been co-introduced.

3. The transformant according to claim 1 or 2, wherein expression of the heterodimer of somatostatin receptor subtype 1 and somatostatin receptor subtype 4 is confirmed by expressing somatostatin receptor subtype 1 and somatostatin receptor subtype 4 which have been each differently labeled in the transformant, and determining a proximity between the different labels by a proximity ligation assay method.

4. The transformant according to any one of claims 1 to 3, for elucidating a signal transduction system involving the heterodimer of somatostatin receptor subtype 1 and somatostatin receptor subtype 4.

5. An isolated heterodimer of somatostatin receptor subtype 1 and somatostatin receptor subtype 4.

6. A method for screening a compound that interacts with a heterodimer of somatostatin receptor subtype 1 and somatostatin receptor subtype 4, comprising selecting the compound that interacts with the heterodimer from test compounds.

7. The method according to claim 6, comprising the following steps (a), (b), and (c):
(a) a step of bringing a test compound into contact with a heterodimer of somatostatin receptor subtype 1 and somatostatin receptor subtype 4;
(b) a step of measuring interaction of the heterodimer with the test compound; and
(c) a step of selecting a compound that interacts with the heterodimer based on the measurement results in (b) above.

8. The method according to claim 7, comprising the following steps (d), (e), and (f) after step (c):
(d) a step of bringing the test compound selected in (c) into contact with a homodimer of somatostatin receptor subtype 1 and/or a homodimer of somatostatin receptor subtype 4;
(e) a step of measuring interaction of the test compound with the homodimer; and
(f) a step of selecting a compound having a weaker interaction than the interaction measured in (b) based on the measurement results in (e) above.

9. The method according to any one of claims 6 to 8, wherein the compound is an agonist or allosteric modulator of the heterodimer of somatostatin receptor subtype 1 and somatostatin receptor subtype 4.

10. The method according to any one of claims 6 to 8, wherein the compound is a compound used to identify an organ, tissue, or cell expressing the heterodimer of somatostatin receptor subtype 1 and somatostatin receptor subtype 4.

11. The method according to any one of claims 6 to 8, which is a method for screening a compound for treating and/or preventing a disease caused by accumulation of amyloid β protein.
